# FASCICULE DE BREVET EUROPEEN

(11) **EP 0 534 855 B1**
(45) Date de publication et mention de la délivrance du brevet: **23.04.1997**
(21) Numéro de dépôt: 92402626.3
(22) Date de dépôt: 24.09.1992
(51) Int. Cl.: C08B 37/00, C12P 19/04

(54) **Polysaccharide, ses applications, son obtention par fermentation, souche de Pseudomonas le produisant**
Polysaccharid, seine Verwendungen, der Fermentationsprozess und der Stamm von Pseudomonas zu seiner Herstellung
Polysaccharide, applications thereof, fermentation process to obtain the same and pseudomonas strain producing it

(30) Priorité: 25.09.1991 FR 9111823
(43) Date de publication de la demande: 31.03.1993
(73) Titulaire: SYSTEMS BIO-INDUSTRIES, F-75008 Paris (FR)
(72) Inventeur: Fontaine, Thierry, F-50810 La Barre de Semilly (FR); Fournet, Bernard, F-59493 Villeneuve D'Asq (FR); Planard, Marie-France, F-50500 Carentan (FR)
(74) Mandataire: Polus, Camille

(56) Documents cités:
- EP-A- 0 001 895
- EP-A- 0 023 397
- EP-A- 0 410 604
- GB-A- 2 058 812

## Description

La présente invention concerne un nouveau polysaccharide, son procédé d'obtention par culture d'un microorganisme du genre Pseudomonas ainsi que ce microorganisme, et les applications de ce polysaccharide comme agent de viscosité, épaississant, gélifiant ou stabilisant de suspensions.

On connaît différentes espèces de microorganismes producteurs de polysaccharides dont les propriétés rhéologiques particulières permettent leur utilisation comme agents de suspension ou comme agents épaississants ou gélifiants des milieux aqueux; le xanthane entre, par exemple, dans la composition d'encres, de peintures, de produits alimentaires ou de médicaments; le scléroglucane est utilisé dans les forages pétroliers.

On a maintenant isolé une souche de microorganismes du genre Pseudomonas, dans un prélèvement de terre de Normandie qui, par culture sur un milieu nutritif classique contenant des sources de carbone et d'azote organique et des sels minéraux, secrète un nouveau polysaccharide dont les propriétés rhéologiques en milieu aqueux sont particulièrement intéressantes, du même type que celles du xanthane mais se manifestant à plus faible concentration.

Selon un premier aspect, l'invention concerne donc une souche bactérienne dénommée PS-5T1, dont un échantillon a été déposé en application du traité de Budapest, le 24 Septembre 1991 sous le numéro I-1145 à la Collection Nationale de Cultures de Microorganismes - Paris, France (CNCM).

Les bactéries PS-5T1 sont Gram négatif; elles ont une forme de bâtonnet de dimension moyenne 0,7 à 8,0 µm sur 1,1 à 1,45 µm.

Sur milieu nutritif gélosé Plate Count Agar, commercialisé par DIFCO, la souche forme des colonies rondes, bombées, jaunes, brillantes, atteignant 2 mm de diamètre après 48 heures à 28°C. Sur milieu MY gélosé (DIFCO), l'aspect des colonies est identique, et leur diamètre augmente si l'incubation se prolonge.

Les propriétés physiologiques et biochimiques des bactéries PS5T₁, déterminées selon les méthodes décrites dans Bergey's Manual, Noel R. KRIEG and al, 1974 et - The Prokaryotes, Mortimer P. STARR, 1981, figurent dans le tableau I.

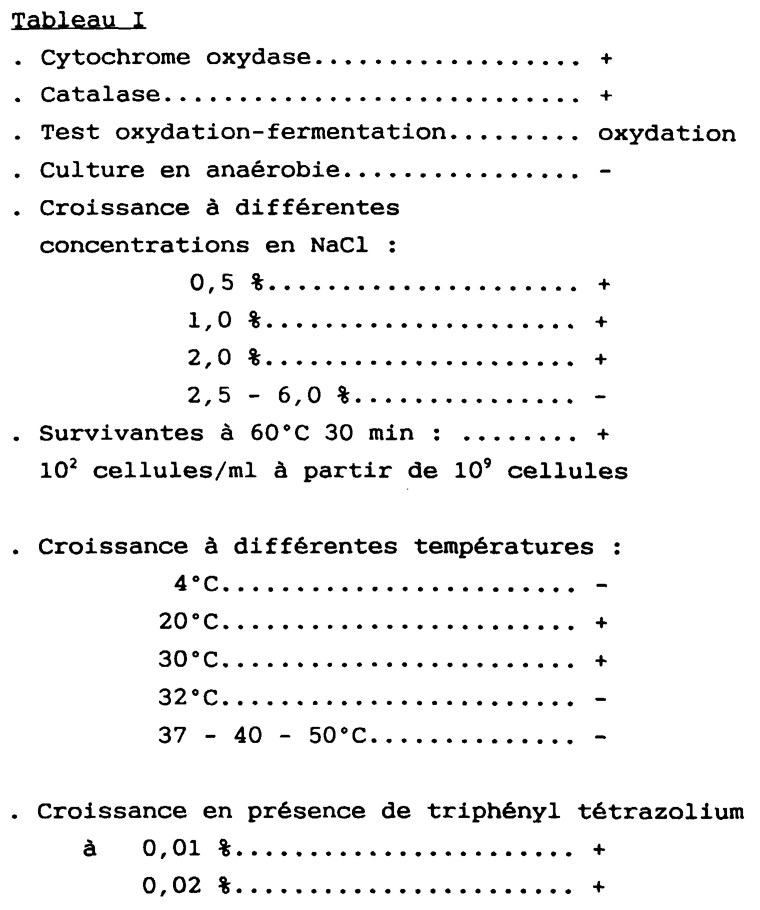

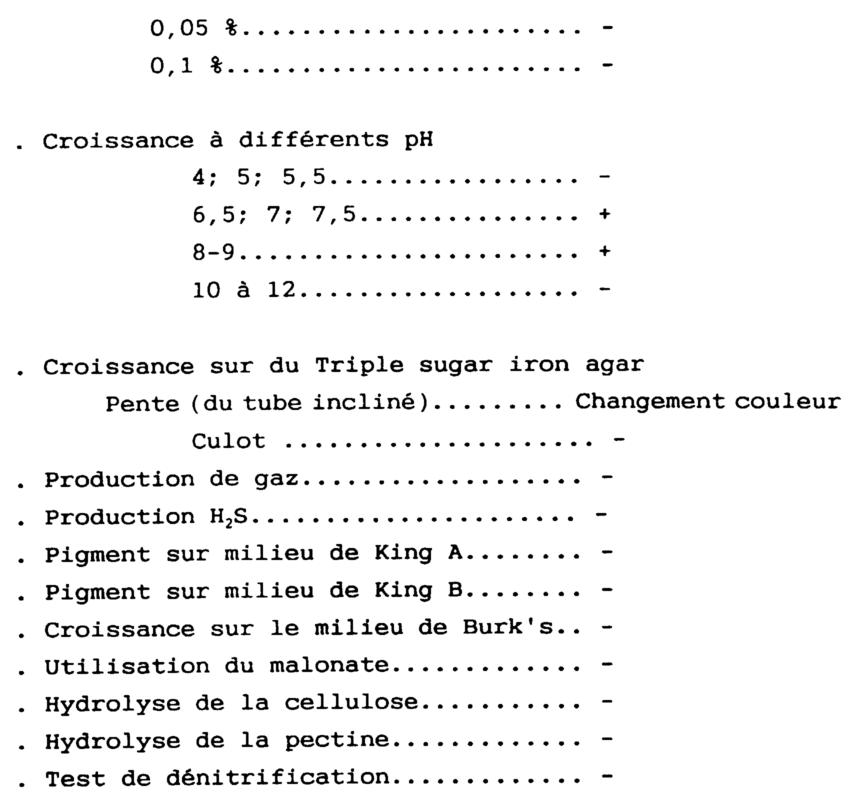

Dans le tableau II sont mentionnés les résultats d'essais enzymatiques classiques effectués en utilisant les ensembles de réactifs commercialisés par la Société API System, la Balme-les-Grottes 38390 - Montalieu Vercieu (FR).

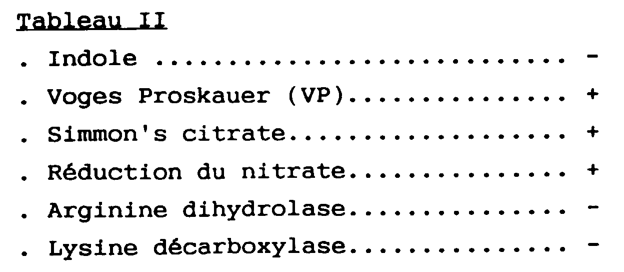

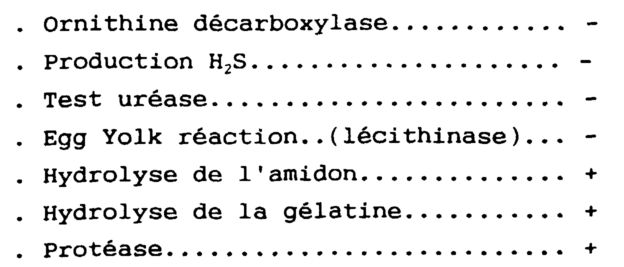

Le polysaccharide selon l'invention peut être obtenu par fermentation de ces bactéries dans un milieu de culture convenable, agité et aéré de façon classique.

Les bactéries PS-5T1 ne se développent pas sur un milieu contenant comme seule source d'azote un sel d'ammonium, mais la source d'azote présente dans le milieu de fermentation peut être de diverses origines protéiques, telles que, par exemple, des extraits de levure, des farines de soja, des eaux de trempage de maïs, de la gélatine, des drèches de distillerie, des peptones. Ces protéines sont introduites à raison de 0,1 % à 1 % (p/v) du milieu liquide aqueux, ce qui correspond à un poids équivalent en azote total de 0,16 à 1,6 g/l environ.

Les bactéries peuvent utiliser un grand nombre d'hydrates de carbone comme source de carbone et par exemple le galactose, le glucose, le mannose, l'amygdaline, la cellobiose, le maltose, l'amidon, le glycogène et le lactose et le milieu nutritif peut contenir un seul de ces composés ou un mélange de ceux-ci. En général, on en introduit de 2 % à 6 % (p/v) dans le milieu aqueux de fermentation.

Parmi les sels minéraux facteurs de croissance utilisables pour la culture des bactéries PS-5TI, on choisira les sels apportant des ions Na, K, NH₄, Ca, Mg, PO₄, SO₄, Cl, CO₃. On introduit aussi de façon classique dans le milieu nutritif, de préférence, des oligo-éléments tels que Cu, Mn, Fe, Zn, à raison de quelques ppm.

La fermentation qui permet de produire le polymère de l'invention, peut être réalisée en milieu agité et aéré à une température comprise entre 20°C et 32°C, et de préférence à 28°C; le pH du milieu est compris entre 6,5 et 9,0 et de préférence vers 7,0 et il est ajusté si nécessaire en cours de fermentation. Une fermentation dure de 30 heures à 80 heures avant que le milieu ne devienne trop épais.

Le polysaccharide est isolé du milieu de fermentation en fin de culture, de préférence, par précipitation; pour cela, on introduit dans le milieu, après sa stérilisation, un solvant miscible à l'eau, dans lequel le polymère est insoluble, tel qu'un alcool comme l'éthanol, le méthanol et de préférence l'isopropanol, ou une cétone, telle que l'acétone; de cette façon, on isole le polysaccharide brut, co-précipité avec des corps cellulaires et des sels; on dit que le polysaccharide est sous forme native.

Il peut être utilisé tel quel ou après purification notamment par dialyse pour éliminer les sels et par reprécipitation à partir d'une solution aqueuse à faible concentration, 1 à 5 g/l ou de préférence 2 à 3 g/l, par addition de l'un des solvants précédemment cités, en général par addition d'isopropanol.

Pour améliorer sa dispersibilité en milieu aqueux on pourra, comme on le fait pour d'autres polysaccharides dont le xanthane, traiter le polymère de l'invention par un dialdéhyde aliphatique, comme le glutaraldéhyde.

Un autre objet de l'invention est le polysaccharide, dénommé 5T1, dans ses différentes formes, qui peut être obtenu par culture des bactéries PS-5T₁, caractérisé en ce qu'il est constitué d'unités répétitives dont le squelette de chacune comporte 2 radicaux de d-mannose, 2 de d-glucose, 1 de d-galactose, 1 d'acide d-glucuronique, 1 de d-xylose, 1 de 1-lyxose et 1 de I-fucose, et dont certains hydroxyles saccharidiques sont éventuellement estérifiés, notamment sous forme d'acétate.

Selon les conditions de culture et d'isolement, le polysaccharide peut présenter différentes formes, c'est-à-dire porter plus ou moins de groupes acétate et pyruvate sur le squelette.

La structure et l'enchaînement de chaque unité du polysaccharide ont été déterminés sur un échantillon de masse sensiblement homogène par des techniques classiques d'analyse, et plus particulièrement, une forme du polysaccharide 5T₁ est constituée d'une succession d'unités nonasaccharidiques dont le squelette de base, sur lequel est greffé un groupe pyruvique, est représenté par la formule développée I suivante dans lequelle R éventuellement présent sur le lyxose représente un groupe en C₁ à C₆ :

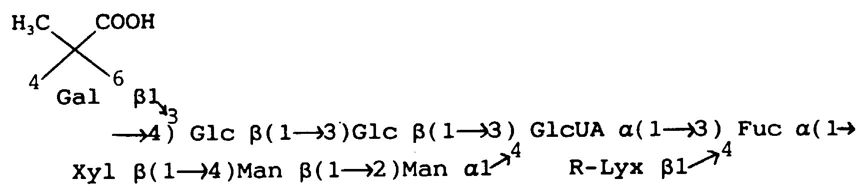

Dans une forme particulière, le polysaccharide 5T₁, est constitué du produit de formule I sur laquelle sont fixés trois groupes acétate.

Les propriétés rhéologiques du nouveau polymère selon l'invention sont particulièrement avantageuses :
- solubilité à température ambiante, non négligeable,
- compatibilité avec le lait,
- il présente un seuil d'écoulement,
- en solution le polymère selon l'invention a une viscosité supérieure à celle du xanthane. La viscosité d'une solution à 1% est stable même en milieu acide, tel que dans l'acide acétique à 20%, ou en présence d'un sel tel que NaCl, Na₂SO₄, CaCl₂, et n'évolue pas lorsqu'on élève la température de la solution de 20°C à 90°C mais disparaît à pH >10,
- en présence d'un cation trivalent, tel que Al³⁺, Fe³⁺, Cr³⁺, en milieu aqueux le polysaccharide 5T₁ gélifie; ainsi, on obtient un gel cohérent avec une solution aqueuse à 0,5% du polymère contenant 50 ppm d'Al³⁺, 100 ppm de Fe³⁺ ou 200 ppm de Cr³⁺.

Il peut être utilisé seul ou en mélange avec les autres agents épaississants et gélifiants connus : carraghénane, xanthane, guar, caroube, alginate, pectine ou la gélatine, pour des applications techniques ou alimentaires. Notamment, on peut l'introduire dans des compositions cosmétiques, pharmaceutiques ou phytosanitaires, dans des colorants pour l'impression des textiles, dans des encres d'imprimerie, des peintures, des ciments et dans les compositions alimentaires telles que les sauces, les crèmes-desserts, les compositions sucrées aux fruits, particulièrement en association avec la pectine, ou les compositions lactées, en association avec le carraghénane.

Dans ce qui suit, on décrit un exemple de préparation du polysaccharide selon l'invention, les méthodes de détermination de sa structure et ses propriétés rhéologiques, et des exemples d'application.

### EXEMPLE 1

Des bactéries Pseudomonas de la souche I-1145 ont été ensemencées sur un milieu gélosé MY. Après 48 heures d'incubation à 28°C, la culture obtenue a été utilisée pour ensemencer 100 ml de bouillon MY dans une fiole et après 24 heures d'incubation à 28°C, on a ensemencé 1000 ml du même bouillon; enfin après 12 heures de culture les bactéries finalement obtenues ont été introduites dans 10 litres d'un milieu nutritif convenant pour la production du polysaccharide 5T₁ composé de : glucose (35 g/l), farine de soja (5 g/l), K₂HPO₄, (3 g/l), MgSO₄, (1 g/l), MnSO₄, (50 µg/l), FeSO₄, (50 µg/l), ZnSO₄, (50 µg/l), CuSO₄, (50 µg/l), la fermentation a été effectuée à 28°C en milieu agité et aéré tandis que le pH du milieu était maintenu à 7. Après 50 heures environ, le milieu a été porté à 80°C pendant 15 min, pour l'opération dite de stérilisation. Après retour à température ambiante, on a introduit dans le milieu 2,5 volumes d'isopropanol pour précipiter le polysaccharide 5T₁.

Après séchage vers 55°C, on a isolé 20 g du polymère natif recherché.

Le polysaccharide ainsi obtenu peut être introduit dans environ 10 litres d'eau distillée, et la suspension filtrée à travers une couche de terre de diatomées; en introduisant dans le filtrat 3 à 5 volumes d'isopropanol, on précipite le polysaccharide dit pur.

### Propriétés du polysaccharide 5T₁

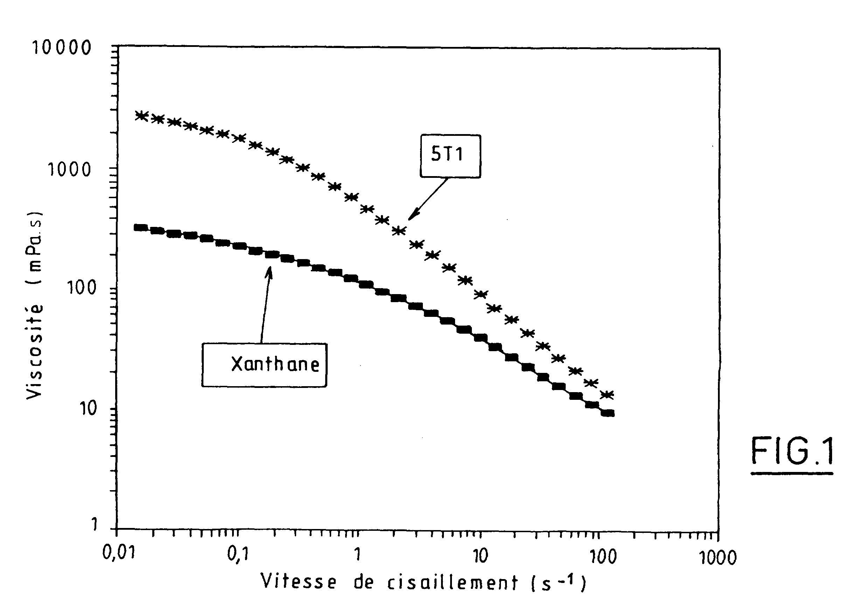

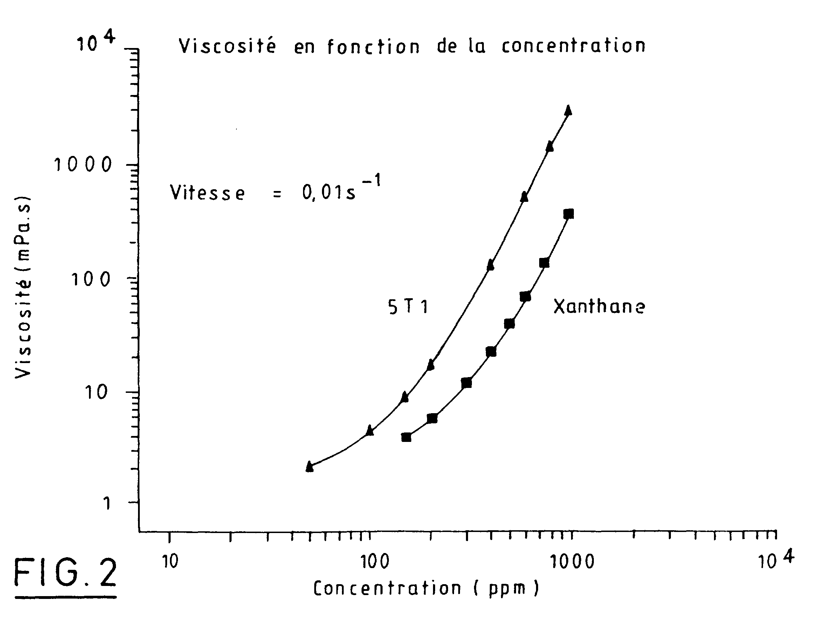

On a effectué des mesures viscosimétriques à 20°C pour un xanthane courant et pour le polysaccharide pur obtenu selon l'exemple ci-dessus, en solution aqueuse en présence de chlorure de potassium (10 g/l), avec un viscosimètre Rhéomat LS 30, commercialisé par la société Contraves (FR), soit à différentes vitesses de cisaillement avec des solutions à 10 g/l, soit à la vitesse de cisaillement 0,01 seconde⁻¹ pour de faibles concentrations. Les courbes obtenues sont représentées figures 1 et 2 en annexe.

Les résultats d'autres mesures effectuées avec un viscosimètre Brookfield, modèle LVF, comportant un mobile référence 2 ou 3 et tournant à 30 tours par minute, pour des solutions de différentes concentrations, contenant éventuellement 10 g/l de chlorure de potassium, figurent tableau III :

**TABLEAU III**

| concentration du polymère (g/100 ml) | KCl | référence mobile | viscosité (mPa) | |
|---|---|---|---|---|
| | | | produit natif | produit purifié |
| 0,5 | - | 3 | 760 | 1200 |
| 0,5 | + | 3 | 1240 | 1640 |
| 0,2 | - | 2 | 190 | 280 |
| 0,2 | + | 2 | 240 | 350 |
| 0,075 | - | 2 | 44 | 62 |
| 0,075 | + | 2 | 46 | 70 |

### Etude structurale

Des fragments saccharidiques ont été obtenus soit par fragmentation avec CH₃OH/HCl à 80°C du polymère éventuellement prétraité et acétylé, soit après hydrolyse par l'acide trifluoroacétique à 100°C, suivie d'une réduction puis d'une acétylation; ils ont été étudiés,en général, par une méthode couplant la chromatographie en phase gazeuse et la spectrométrie de masse.

Le polysaccharide 5T₁ purifié obtenu dans les conditions de l'exemple précédent a été prétraité :
- par le lithium dans l'éthylènediamine pour dégrader les acides uroniques,
- par le métaperiodate de sodium avant et après saponification, ce qui ne décompose que xylose, lyxose et mannose,
- par CrO₃, dans l'acide acétique, ce qui détruit les saccharides peracétylés en configuration anomérique équatoriale,
- par une base forte en milieu anhydre (LiCH₃SC⁻ dans le diméthylsulfoxyde) qui dégrade, par β-élimination, les acides uroniques des polysaccharides perméthylés.

La présence de substituants acétiques a été mise en évidence par chromatographie liquide sur colonne d'exclusion d'ions après l'hydrolyse du polysaccharide 5T₁. Le groupe pyruvique, vraisemblablement lié au galactose par une liaison acétal sur les hydroxyles 4 et 6, a été mis en évidence après méthanolyse du polysaccharide et de son dérivé perméthylé. Le groupe R présent sur le lyxose n'a pas été caractérisé.

### EXEMPLE 2

### Stabilisation de ciments artificiels :

Après 8 jours de conservation à température ambiante, ou 4 jours à 50°C, une composition aqueuse contenant 50% en poids de ciment de type Portland et 1% de polysaccharide PS-5T1, reste homogène, malléable, et présente seulement un léger durcissement en surface.

### EXEMPLE 3

### Crème-dessert :

On prépare des crèmes ayant la composition suivante :

| | crème vanille | crème chocolat |
|---|---|---|
| - Poudre de lait écrémé | 3,5 % en poids | 1,5 % |
| - Sucre | 10 % | 12 % |
| - Amidon | 1,7 % | 1,7 % |
| - Cacao | -- | 3 % |
| - Arôme vanille | 0,3 % | -- |
| - Stabilisant * | 0,2 % | 0,18% |
| - Lait entier | q.s.p 100 | q.s.p 100 |

| | | |
|---|---|---|
| * composé de 65% (p/p) de carraghénane et 35% de PS-5T1. | | |

Les ingrédients prémélangés à sec sont dispersés dans le lait froid. Après hydratation pendant 15 minutes, le mélange est passé sur un échangeur à plaques et subit le traitement suivant :
- pasteurisation à 90°C
- homogénéisation à 50 kg/cm²
- stérilisation à 130°C
- refroidissement à 65°C.
Il est ensuite conditionné.

Dans les deux cas, la texture finale des crèmes est épaisse, lisse, brillante et sans bulles d'air.

Des crèmes témoins contenant comme stabilisant uniquement du carraghénane ou du xanthane ont une texture et un aspect moins homogènes et présentent des bulles ; en outre, les crèmes selon l'invention s'écoulent mieux dans les appareils.

### EXEMPLE 4

### Sauces salades :

On prépare des sauces salades acides ayant la composition suivante :
- Eau de ville 25,05 % en poids
- Vinaigre blanc 8° 15,90 %
- Concentré de tomate (28%) 3,60 %
- Sel 4,20 %
- Poivre 0,60 %
- Sucre 10,40 %
- Huile de soja 40,00 %
- Stabilisant * 0,25 %

* stabilisant : xanthane ou polysaccharide PS-5T1.

On disperse sous agitation le stabilisant dans l'huile (mélange 1). On disperse dans l'eau sous agitation les autres ingrédients secs (mélange 2). On mélange le vinaigre avec le concentré de tomates (mélange 3).

On introduit successivement les mélanges 1, 2 puis 3 dans un homogénéisateur Herbort, puis on émulsionne pendant 3 minutes sous vide avant de conditionner et stocker à température ambiante.

Les viscosités des sauces ont été mesurées avant le conditionnement :

| | Viscosité Brookfield | Temps d'écoulement Bostwick (14 cm) |
|---|---|---|
| Xanthane | 2 450 cps | 51 secondes |
| PS-5T1 | 2 800 cps | 94 secondes |

On constate que la sauce selon l'invention est plus stable durant la conservation.

### EXEMPLE 5

### Pâtes dentifrices :

On prépare des dentifrices de la composition suivante :
- Saccharinate de Na 0,2 % en poids
- Benzoate de Na 0,5 %
- Stabilisant * 1 %
- Pyrophosphate de Na 0,25 %
- Glycérine 22 %
- Phosphate de calcium 53 %
- Laurylsulfate de Na 1,5 %
- Arôme q.s.
- Eau q.s.p. 100

* Stabilisant : polysaccharide PS-5T1 ou xanthane.

On disperse d'abord le saccharinate, le benzoate, le PS-5T1 et le pyrophosphate dans la glycérine ; l'eau est ajoutée après 5 minutes d'agitation, puis la solution est portée au bain-marie à 75°C pendant 15 minutes. Elle est ensuite versée sur le phosphate de calcium sous agitation ; après 10 minutes, on verse le laurylsulfate et l'arôme.

Les dentifrices selon l'invention ont une belle texture avec un aspect lisse, et ils présentent une viscosité stable et supérieure d'environ 30% à celle obtenue avec un témoin, stabilisé avec du xanthane.

## Revendications

1. Polysaccharide caractérisé en ce qu'il est constitué d'unités répétitives saccharidiques qui ont un squelette comportant 2 radicaux de d-mannose, 2 de d-glucose, 1 de d-galactose, 1 d'acide d-glucuronique, 1 de d-xylose, 1 de l-lyxose et 1 de l-fucose.

2. Polysaccharide selon la revendication 1, caractérisé en ce qu'il comprend au moins un radical d'acide pyruvique greffé par unité nonasaccharidique.

3. Polysaccharide selon l'une des revendications 1 et 2, caractérisé en ce que certains hydroxyles saccharidiques sont estérifiés, sous forme d'acétate.

4. Polysaccharide selon l'une des revendications 2 et 3, caractérisé en ce qu'il est constitué par une succession d'unités répétitives dont le squelette sur lequel est greffé un groupe pyruvique répond à la formule I 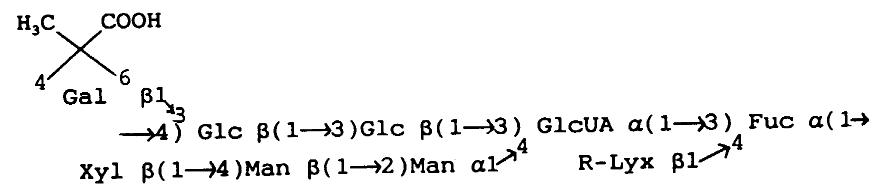 dans laquelle R éventuellement présent est un groupe en C₁ à C₆.

5. Polysaccharide selon la revendication 4, caractérisé en ce que 3 des hydroxyles saccharidiques, par unité, sont sous forme acétate.

6. Procédé d'obtention d'un polysaccharide selon l'une des revendications 1 à 5, qui consiste à cultiver des bactéries de la souche CNCM N° I-1145 dans un milieu nutritif et à isoler le polysaccharide produit lors de la fermentation.

7. Procédé selon la revendication 6, caractérisé en ce que le milieu de fermentation aéré est à une température comprise entre 20°C et 32°C, un pH compris entre 6,5 et 9 et qu'il contient une source de carbone, une source d'azote organique et des facteurs de croissance.

8. Polysaccharide obtenu selon le procédé de l'une des revendications 6 et 7, à l'état natif.

9. Souche de bactérie Pseudomonas déposée à la CNCM sous le N° I-1145.

10. Utilisation d'un polysaccharide selon l'une des revendications 1 à 5 et 8 pour l'obtention de gels aqueux en présence de cations trivalents.

11. Utilisation d'un polysaccharide selon l'une des revendications 1 à 5 et 8 comme agent de viscosité, épaississant ou stabilisant de suspensions.

## Patentansprüche

1. Polysaccharid, dadurch gekennzeichnet, daß es gebildet ist aus sich wiederholenden Saccharideinheiten, welches ein Skelett aufweist, das 2 D-Manose-, 2 D-Glucose-, 1 D-Galactose-, 1 D-Glucoronsäure-, 1 D-Xylose-, 1 L-Lyxose- und 1 L-Fucosereste enthält.

2. Polysaccharid nach Anspruch 1, dadurch gekennzeichnet, daß das Polysaccharid wenigstens einen über eine Nonasaccharideinheit aufgepfropften Brenztraubensäurerest enthält.

3. Polysaccharid nach einem der Ansprüche 1 und 2, dadurch gekennzeichnet, daß verschiedene Saccharidhydroxylgruppen als Acetat verestert sind.

4. Polysaccharid nach einem der Ansprüche 2 und 3, dadurch gekennzeichnet, daß das Polysaccharid gebildet ist durch eine Aufeinanderfolge von sich wiederholenden Einheiten, auf deren Skelett eine Pyruvatgruppe der Formel I 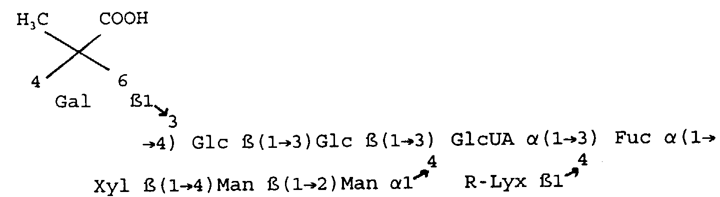 aufgepropft ist, in der das gegebenenfalls vorkommende R eine C₁ bis C₆-Gruppe ist.

5. Polysaccharid nach Anspruch 4, dadurch gekennzeichnet, daß drei der Saccharidhydroxylgruppen je Einheit als Acetat vorliegen.

6. Verfahren zur Herstellung eines Polysaccharids nach den Ansprüchen 1 bis 5, dadurch gekennzeichnet, daß das Verfahren aus dem Kultivieren von Bakterien des Stamms CNCM Nr. I-1145 in einem Nährmedium und aus dem Isolieren des während der Fermentierung erzeugten Polysaccharids besteht.

7. Verfahren nach Anspruch 6, dadurch gekennzeichnet, daß das belüftete Fermentationsmilieu eine Temperatur zwischen 20°C und 32°C, einen pH-Wert zwischen 6,5 und 9 aufweist und daß es eine Kohlenstoffquelle, eine organische Stickstoffquelle und Wachstumfaktoren enthält.

8. Polysaccharid, erhalten nach dem Verfahren nach einem der Ansprüche 6 und 7 im nativen Zustand.

9. Bakterienstamm Pseudomonas, hinterlegt als CNCM unter Nr. I-1145.

10. Verwendung eines Polysaccharids nach einem der Ansprüche 1 bis 5 und 8 zur Herstellung eines wässrigen Gels in Gegenwart von dreiwertigen Kationen.

11. Verwendung eines Polysaccharids nach einem der Ansprüche 1 bis 5 und 8 als Viskositäts-, Verdickungsoder Stabilisierungsmittel für Suspensionen.

## Claims

1. Polysaccharide characterised in that it is formed of repetitive saccharide units which have a skeleton including 2 d-mannose radicals, 2 d-glucose radicals, 1 d-galactose radical, 1 d-glucuronic acid radical, 1 d-xylose radical, 1 l-lyxose radical and 1 I-fucose radical.

2. Polysaccharide as claimed in Claim 1, characterised in that it comprises at least one pyruvic acid radical grafted by non-saccharide unit.

3. Polysaccharide as claimed in one of Claims 1 and 2, characterised in that certain saccharide hydroxyls are esterified, in the form of acetate.

4. Polysaccharide as claimed in one of Claims 2 and 3, characterised in that it is formed by a succession of repetitive units of which the skeleton on which a pyruvic group is grafted complies with formula I 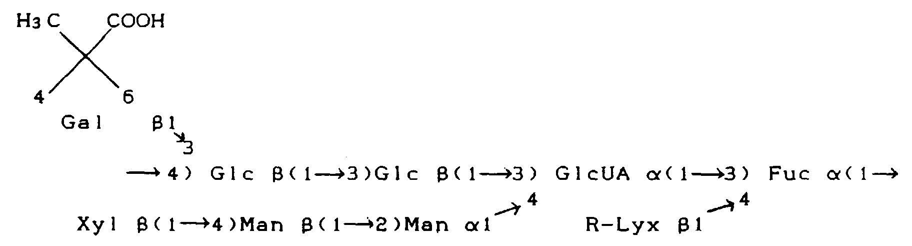 in which R which may be present has a C₁ to C₆ group.

5. Polysaccharide as claimed in Claim 4, characterised in that 3 of the saccharide hydroxyls per unit are in acetate form.

6. Method of obtaining a polysaccharide as claimed in one of Claims 1 to 5, consisting of culturing bacteria of the strain CNCM N° I-1145 in a nutritive medium and isolating the polysaccharide produced during the fermentation.

7. Method as claimed in Claim 6, characterised in that the aerated fermentation medium is at a temperature between 20°C and 32°C and a pH between 6.5 and 9 and that it contains a source of carbon, a source of organic nitrogen and growth factors.

8. Polysaccharide obtained using the method as claimed in one of Claims 6 and 7, in the native state.

9. Strain of Pseudomonas bacterium deposited with the CNCM under N° I-1145.

10. Use of a polysaccharide as claimed in one of Claims 1 to 5 and 8 for obtaining aqueous gels in the presence of trivalent cations.

11. Use of a polysaccharide as claimed in one of Claims 1 to 5 and 8 as a viscosity agent, thickener or stabiliser of suspensions.
